# EUROPEAN PATENT APPLICATION

(11) **EP 4 495 234 A1**
(43) Date of publication of application: **22.01.2025**
(21) Application number: 23771132.0
(22) Date of filing: 17.03.2023
(51) Int. Cl.: C12N 9/88, C12N 15/77, C12P 13/06, C12R 1/15

(54) **VARIANT OF ASPARTATE 1-DECARBOXYLASE DERIVED FROM T. CASTANEUM AND MICROORGANISMS INCLUDING SAME**

(30) Priority: 18.03.2022 KR 20220034233
(71) Applicant: CJ CheilJedang Corporation, Seoul 04560 (KR)
(72) Inventor: JANG, Yeon-Jae, Seoul 04560 (KR); YANG, Sungjae, Seoul 04560 (KR); SHIN, Kwang Soo, Seoul 04560 (KR); SO, Yee-Seul, Seoul 04560 (KR); LEE, Kwang Woo, Seoul 04560 (KR)
(74) Representative: Reddie & Grose LLP
(86) International application number: PCT/KR2023/003572
(87) International publication number: WO 2023/177253

(57) **Abstract**

The present application provides: a variant polypeptide having an activity of aspartate 1-dicarboxylase; a microorganism including same; a composition for producing beta-alanine or beta-alanine-derived compounds, the composition comprising the microorganism; and a method for producing beta-alanine or beta-alanine-derived compounds, the method comprising a step for culturing the microorganism.

## Description

### [TECHNICAL FIELD]

### Cross-reference to related application(s)

The present application claims the benefit of the priority based on Korean Patent Application No. 10-2022-0034233 filed on March 18, 2022, and the entire contents disclosed in the document of the corresponding Korean patent application are incorporated as a part of the present description.

Provided are a variant polypeptide of aspartate 1-dicarboxylase derived from T. *castaneum*, a microorganism including the same, a composition for producing beta-alanine or beta-alanine-derived compounds comprising the microorganism, and a method for producing beta-alanine or beta-alanine-derived compounds comprising a step for culturing the microorganism.

### [BACKGROUND ART]

Beta-alanine (β-alanine) is a naturally occurring β-amino acid in which an amino group is combined to the β carbon. Beta-alanine is found naturally in foods such as poultry, meat and fish, and is used to synthesize carnosine in muscles.

Beta-alanine-derived compounds, such as beta-alanine and pantothenic acid, are one of commercially important substances applied in various industrial fields such as health supplementary foods, foods, pharmaceuticals, animal feeds, and the like.

Accordingly, development of a microorganism having an advantageous effect in biotechnologically preparing beta-alanine and/or beta-alanine-derived compounds, and a technology for preparing beta-alanine and/or beta-alanine-derived compounds using the same with high efficiency is required.

### [PRIOR ART]

### [PATENT DOCUMENT]

(Patent document 1) U.S. Patent No. 7718205

### [DISCLOSURE]

### [TECHNICAL PROBLEM]

The present application provides a variant polypeptide having an activity of aspartate 1-decarboxylase, in which the amino acid corresponding to the 139th residue of the amino acid sequence of SEQ ID NO: 51 is substituted with another amino acid.

The present application provides a polynucleotide encoding the variant polypeptide.

The present application provides a recombinant vector comprising the polynucleotide.

The present application provides a microorganism comprising the variant polypeptide and/or a polynucleotide encoding the same.

The present application provides a composition for producing beta-alanine or beta-alanine-derived compounds comprising a microorganism comprising the variant polypeptide and/or a polynucleotide encoding the same.

The present application provides a method for producing beta-alanine or beta-alanine-derived compounds, comprising culturing a microorganism comprising the variant polypeptide and/or a polynucleotide encoding the same.

The present application provides a use of the microorganism for using in production of beta-alanine and/or beta-alanine-derived compounds.

The present application provides a use of the microorganism for using in preparation of a composition for producing beta-alanine and/or beta-alanine-derived compounds.

### [TECHNICAL SOLUTION]

In the present application, a variant polypeptide having activity of aspartate 1-decarboxylase (or PanD protein) capable of improving production ability of beta-alanine and/or beta-alanine-derived compounds, or a recombinant microorganism (strain) with excellent production ability of beta-alanine and/or beta-alanine-derived compounds in which the variant polypeptide is introduced is to be provided.

One aspect of the present application to achieve the above object may provide a variant polypeptide having activity of aspartate 1-decarboxylase. The variant polypeptide may comprise a substitution of the amino acid corresponding to the 139^{th} residue of the amino acid of SEQ ID NO: 51 with another amino acid.

Another embodiment provides a polynucleotide encoding (or coding) the polypeptide.

Other embodiment provide a recombinant vector comprising the polynucleotide. The recombinant vector may be used as an expression vector of the polypeptide.

Other embodiment provides a microorganism comprising at least one selected from the group consisting of the polypeptide, a polynucleotide encoding the polypeptide and a recombinant vector comprising the polynucleotide. The microorganism may be a microorganism producing beta-alanine and/or beta-alanine-derived compounds.

Other embodiment provides a composition for producing beta-alanine and/or beta-alanine-derived compounds comprising the microorganism.

Other embodiment provides a method for producing beta-alanine and/or beta-alanine-derived compounds comprising culturing the microorganism.

Hereinafter, it will be described in more detail.

### Polypeptide

One aspect of the present application provides a variant polypeptide having activity of aspartate 1-decarboxylase (or PanD protein). The variant polypeptide having activity of aspartate 1-decarboxylase may be a variant polypeptide in which a mutation that at least one amino acid residue is substituted, deleted, or inserted into the aspartate 1-decarboxylase protein.

The protein subjected to mutation introduction of the present application may be a protein having activity of aspartate 1-decarboxylase. The sequence of the protein may be obtained from NCBI, a known database, and specifically, it may consist of the amino acid sequence of SEQ ID NO: 51 or comprise the amino acid sequence of SEQ ID NO: 51 and have activity of aspartate 1-decarboxylase, but not limited thereto. In other words, it does not exclude addition of meaningless sequences before and after the amino acid sequence of SEQ ID NO: 51 or mutation that may occur naturally, or silent mutation thereof, and as long as it has the same or corresponding activity as the protein comprising the amino acid sequence of SEQ ID NO: 51, it may correspond to the protein subjected to mutation introduction of the present application. For example, the protein subjected to mutation introduction of the present application may be a protein consisting of the amino acid sequence of SEQ ID NO: 51, or the amino acid sequence having homology or identity of 80%, 85%, 90%, 95%, 96%, 97%, 98%, or 99% or more thereto. In addition, as long as it is an amino acid sequence that has such homology or identity and exhibits efficacy corresponding to the protein, a protein having amino acid sequences in which some sequences are deleted, modified, substituted, or added is also included within the scope of the protein subjected to mutation of the present application.

In one embodiment, the variant polypeptide having activity of aspartate 1-decarboxylase may be one that the amino acid corresponding to the 139th residue of the amino acid sequence of SEQ ID NO: 51 is substituted with another amino acid, that is, an amino acid which is selected from the group consisting of tryptophan (Trp, W), histidine (His, H), tyrosine (Tyr, T), alanine (Ala, A), cysteine (Cys, C), proline (Pro, P), serine (Ser, S), leucine (Leu, L), isoleucine (Ile, I), arginine (Arg, R), lysine (Lys, K), valine (Val, V), methionine (Met, M), aspartic acid (Asp, D), glutamic acid (Glu, E), glycine (Gly, G), asparagine (Asn, N), glutamine (Gln, Q), and tyrosine (Tyr, Y), and is different from the original amino acid (phenylalanine, Phe, F). In one specific embodiment, the variant polypeptide having activity of aspartate 1-decarboxylase may be one that the amino acid corresponding to the 139th residue of the amino acid sequence of SEQ ID NO: 51 is substituted with a basic amino acid (arginine, histidine, or lysine), an acidic amino acid (aspartic acid or glutamic acid), a polar amino acid (serine, threonine, asparagine, or glutamine), alanine, valine, isoleucine, leucine, tyrosine, or glycine. In one specific embodiment, the variant polypeptide having activity of aspartate 1-decarboxylase may be one that the amino acid corresponding to the 139th residue of the amino acid sequence of SEQ ID NO: 51 is substituted with threonine, alanine, serine, leucine, isoleucine, valine, aspartic acid, glycine, histidine, or tyrosine. In one specific embodiment, the variant polypeptide having activity of aspartate 1-decarboxylase may be one that the amino acid corresponding to the 139th residue of the amino acid sequence of SEQ ID NO: 51 is substituted with tyrosine. In one specific embodiment, the variant of aspartate 1-decarboxylase may consist of at least one amino acid sequence selected from SEQ ID NO: 52 to SEQ ID NO: 61, or comprise at least one amino acid sequence selected from SEQ ID NO: 52 to SEQ ID NO: 61.

The variant polypeptide of the present application may have a characteristic that increases production ability of beta-alanine and/or beta-alanine-derived compounds, compared to a wild-type polypeptide having activity of aspartate 1-decarboxylase.

It is obvious that even if sone amino acid sequences except the amino acid corresponding to the 139^{th} amino acid residue of the amino acid sequence of SEQ ID NO: 51 is deleted, modified, substituted, or added, in the variant polypeptide, as long as it shows the activity of aspartate 1-decarboxylase, it is included in the variant polypeptide of the present application.

For example, it is a case with addition or deletion of a sequence which does not change the function of the variant polypeptide of the present application at the N-terminus, C-terminus, and/or inside of the amino acid sequence, mutation that may occur naturally, silent mutation or conservative substitution.

The "conservative substitution" means substituting one amino acid with another amino acid having similar structural and/or chemical properties. Such amino acid substitution may generally occur based on similarity in polarity, charge, solubility, hydrophobicity, hydrophilicity, and/or amphipathic nature of a residue. Commonly, the conservative substitution may have little effect or have no effect on the activity of the protein or polypeptide.

In other words, in the variant polypeptide of the present application, the amino acid corresponding to the 139th residue of the amino acid sequence of SEQ ID NO: 51 may be substituted with another amino acid, and it may have or comprise an amino acid sequence having homology or identity of 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.3% or more, 99.5% or more, 99.7% or more, or 99.9% or more to the amino acid sequence of SEQ ID NO: 51, or consist of, or essentially consist of an amino acid sequence with homology or identity of 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.3% or more, 99.5% or more, 99.7% or more, or 99.9% or more to the amino acid sequence of SEQ ID NO: 51, but not limited thereto. In addition, in one embodiment, the variant polypeptide of the present application may have or comprise a sequence in which the amino acid corresponding to the 139th residue of the amino acid sequence of SEQ ID NO: 51 is substituted with another amino acid in an amino acid sequence having homology or identity of at least 70%, 75%, 80%, 85%, 90%, 95%, 96%, 97%, 98%, 99%, 99.5%, 99.7% or 99.9% to SEQ ID NO: 51, or consist of, or essentially consist of a sequence in which the amino acid corresponding to the 139th residue of the amino acid sequence of SEQ ID NO: 51 is substituted with another amino acid in an amino acid sequence having homology or identity of at least 70%, 75%, 80%, 85%, 90%, 95%, 96%, 97%, 98%, 99%, 99.5%, 99.7% or 99.9% to SEQ ID NO: 51, but not limited thereto.

In one embodiment, the variant polypeptide may have sequence homology or sequence identity of 70% or more and less than 100%, 75% or more and less than 100%, 80% or more and less than 100%, 85% or more and less than 100%, 90% or more and less than 100%, 95% or more and less than 100%, 96% or more and less than 100%, 97% or more and less than 100%, 98% or more and less than 100%, 98.2% or more and less than 100%, 98.4% or more and less than 100%, 98.6% or more and less than 100%, 98.8% or more and less than 100%, 98.9% or more and less than 100%, 99% or more and less than 100%, 99.1 % or more and less than 100%, 99.3% or more and less than 100%, 99.5% or more and less than 100%, 99.7% or more and less than 100% or 99.9% or more and less than 100% to the amino acid sequence of SEQ ID NO: 51, or comprise the sequence, or consist of, or essentially consist of the sequence, but not limited thereto.

In the present application, the term, "variant polypeptide" refers to a polypeptide which is different from the amino acid sequence before mutation of the variant polypeptide by conservative substitution and/or modification of at least one amino acid, but maintains functions or properties.

This variant polypeptide may be generally identified by modifying at least one amino acid of the amino acid sequence of the polypeptide, and evaluating characteristics of the modified polypeptide. In other words, the ability of the variant polypeptide may be increased, or unchanged, or reduced compared to the polypeptide before mutation. In addition, some of the variant polypeptide may include a variant polypeptide in which at least one part such as an N-terminal leader sequence or transmembrane domain is removed. Another variant polypeptide may include a variant polypeptide in which a part is removed from the N- and/or C-terminus of the mature protein. The term, "variant polypeptide" may be interchangeably used with terms such as mutated, modified, variant polypeptide, mutated protein, mutation and variant, and the like (as English expressions, modification, modified polypeptide, modified protein, mutant, mutein, divergent, variant, etc.), and as long as it is a term used as a mutated meaning, it is not limited thereto. For the purpose of the present application, the variant polypeptide may be a polypeptide comprising an amino acid sequence in which the amino acid corresponding to the 139h residue from the N-terminus in the amino acid sequence of SEQ ID NO: 51 is substituted with another amino acid.

In addition, the variant polypeptide may comprise deletion or addition of amino acids having minimal effect on the characteristics and secondary structure of the polypeptide. For example, at the N-terminus of the variant polypeptide, a signal (or leader) sequence involved in protein translocation co-translationally or post-translationally can be conjugated. In addition, the variant polypeptide may be conjugated with another sequence or linker to enable confirmation, purification, or synthesis.

### Polynucleotide

Another aspect provides a polynucleotide encoding the variant polypeptide.

In the present application, the term, "polynucleotide" means a DNA or RNA strand of a certain length or more, which is a polymer of nucleotides in which nucleotide monomers are connected in a long chain by covalent bonds.

The polynucleotide of the present application may encode a variant polynucleotide, in which the amino acid corresponding to the 139th residue in the amino acid sequence of SEQ ID NO: 51 is substituted with another amino acid. Otherwise, the polynucleotide of the present application may comprise a base sequence encoding the variant polypeptide, in which the codon corresponding to the 415th to 417th positions in the nucleotide sequence of SEQ ID NO: 62 is substituted with a codon encoding anther amino acid. As one embodiment of the present application, the polynucleotide of the present application may comprise a base sequence encoding the amino acid sequence described in any one SEQ ID NO selected from SEQ ID NO: 52 to SEQ ID NO: 61. As one embodiment of the present application, the polynucleotide of the present application may have or comprise any one sequence selected from SEQ ID NO: 63 to SEQ ID NO: 72. In addition, the polynucleotide of the present application may consist of, or essentially consist of any one sequence selected from SEQ ID NO: 63 to SEQ ID NO: 72.

The polynucleotide of the present application may have various modifications in a coding region within a range that does not change the amino acid sequence of the variant polypeptide of the present application, in consideration of codon degeneracy or preferred codons in an organism intended to express the variant polypeptide of the present application. Specifically, the polynucleotide of the present application may have or comprise a base sequence having homology or identity of 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to any one sequence selected from SEQ ID NO: 63 to SEQ ID NO: 72, or may consist of or essentially consist of a base sequence having homology or identity of 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to any one sequence selected from SEQ ID NO: 63 to SEQ ID NO: 72, but not limited thereto.

In one embodiment, in the sequence having the homology or identity, the codon encoding the amino acid corresponding to the 139^{th} position in SEQ ID NO: 51, may be one of codons encoding tryptophan, histidine, tyrosine, alanine, cysteine, proline, serine, leucine, isoleucine, arginine, lysine, valine, methionine, aspartic acid, glutamic acid, glycine, asparagine, glutamine, or tyrosine. In one specific embodiment, in the sequence having the homology or identity, the codon encoding the amino acid corresponding to the 139^{th} position in SEQ ID NO: 51, may be one of codons encoding a basic amino acid (arginine, histidine, or lysine), an acidic amino acid (aspartic acid or glutamic acid), a polar amino acid (serine, threonine, asparagine, or glutamine), alanine, valine, isoleucine, leucine, tyrosine, or glycine. In one specific embodiment, in the sequence having the homology or identity, the codon encoding the amino acid corresponding to the 139^{th} position in SEQ ID NO: 51, may be one of codons encoding threonine, alanine, serine, leucine, isoleucine, valine, aspartic acid, glycine, histidine, or tyrosine. In one specific embodiment, in the sequence having the homology or identity, the codon encoding the amino acid corresponding to the 139^{th} position in SEQ ID NO: 51, may be one of codons encoding tyrosine.

In addition, the polynucleotide of the present application may be included without limitation, as long as it is a probe which can be prepared from a known gene sequence, for example, a sequence which can be hybridized under a stringent condition with a complementary sequence to all or a part of the polynucleotide sequence of the present application. The "stringent condition" refers to a condition that enables specific hybridization between polynucleotides. This condition is specifically described in the document (See J. Sambrook et al.,Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). For example, a condition in which polynucleotides having high homology or identity with each other, polynucleotides having homology or identity of 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more, are hybridized, and polynucleotides having homology or identity lower than that with each other are not hybridized, or a condition of washing at a salt concentration and temperature corresponding to 60°C, 1×SSC, 0.1% SDS, specifically, 60°C, 0.1×SSC, 0.1% SDS, more specifically, 68°C, 0.1×SSC, 0.1% SDS, once, specifically, twice to 3 times, which is a washing condition of common southern hybridization may be listed.

Hybridization requires that two nucleic acids have complementary sequences, although mismatches between bases may be possible depending on the stringency of hybridization. The term, "complementary" is used to describe the relationship between nucleotide bases capable of hybridization to each other. For example, with respect to DNA, adenine is complementary to thymine and cytosine is complementary to guanine. Accordingly, the polynucleotide of the present application may also comprise not only a substantially similar nucleic acid sequence but also an isolated nucleic acid fragment complementary to the entire sequence.

Specifically, the polynucleotide having homology or identity to the polynucleotide of the present application may be detected using a hybridization condition including a hybridization step at a Tm value of 55 °C and using the aforementioned condition. In addition, the Tm value may be 60 °C, 63 °C or 65 °C, but not limited thereto, and may be appropriately adjusted according to the purpose by those skilled in the art.

The appropriate stringency for hybridizing the polynucleotide depends on the length of the polynucleotide and the degree of complementarity, and variables are well known in the art (for example, J. Sambrook et al., supra).

In the present description, that a polynucleotide (may be interchangeably used with "gene") or polypeptide (may be interchangeably used with "protein") "comprises a specific nucleic acid sequence or amino acid sequence or consists of or is expressed as a specific nucleic acid sequence or amino acid sequence" may mean that the polynucleotide or polypeptide essentially comprises the specific nucleic acid sequence or amino acid sequence, and it may be interpreted to comprise a "substantially equivalent sequence" in which a meaningless mutation (deletion, substitution, modification, and/or addition) is applied to the specific nucleic acid sequence or amino acid sequence within the range of maintaining the original functions and/or desired functions of the polynucleotide or polypeptide (or not to exclude the meaningless mutation).

In the present description, the term 'homology' or 'identity' refers to a degree of similarity between two given amino acid sequences or base sequences and may be expressed as a percentage. The terms, homology and identity may be often used interchangeably.

The sequence homology or identity of the conserved polynucleotide or polypeptide may be determined by a standard array algorithm, and a default gap penalty established by a used program may be used together. Substantially, a homologous or identical sequence may be generally hybridized to the entire or a part of a sequence under a medium or high stringent condition. It is obvious that the hybridization also includes hybridization with a polynucleotide containing a common codon or codon considering codon degeneracy in the polynucleotide.

Whether any two polynucleotide or polypeptide sequences have homology, similarity or identity, may be determined, for example, using a default parameter such as Pearson et al (1988) [Proc. Natl. Acad. Sci. USA 85]: 2444 using a known computer algorithm such as "FASTA" program. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version) (including GCG program package (Devereux, J., et al, NucleicAcids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, using BLAST, or ClustalW of National Center for Biotechnology Information Database, the homology, similarity or identity may be determined.

The homology, similarity, or identity of the polynucleotide or polypeptide may be determined by compared sequence information using for example, GAP computer program such as Needleman et al. (1970), J Mol Biol. 48:443, for example, known in Smith and Waterman, Adv. Appl. Math (1981) 2:482. In summary, the GAP program may be defined as a value of dividing a total number of symbols in the shorter of two sequences by the number of similar aligned symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

In the present description, the term "corresponding to" refers to an amino acid residue at a position listed in a polypeptide, or an amino acid residue similar to, identical to, or homologous to a residue listed in a polypeptide. Confirming the amino acid at the corresponding position may be determining a specific amino acid of a sequence that refers to a specific sequence. "Corresponding region" used in the present application generally refers to a position at a similar or corresponding position in a related protein or reference protein.

For example, any amino acid sequence is aligned with SEQ ID NO: 52, and based on this, each amino acid residue of the amino acid sequence may be numbered by referring to the number position of the amino acid residue corresponding to the amino acid residue of SEQ ID NO: 52. For example, the sequence alignment algorithm described in the present application may confirm a position of an amino acid, or a position where a modification such as substitution, insertion, or deletion or the like occurs, compared to a query sequence (also referred to as a "reference sequence").

In this alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277), and the like may be used, but not limited thereto, and a sequence alignment program known in the art, pairwise sequence comparison algorithm and the like may be appropriately used.

In the present description, the term, "enhancement" of the polypeptide activity, means that the activity of the polypeptide is increased compared to the intrinsic activity. The enhancement may be interchangeably used with terms such as activation, up-regulation, overexpression, increase and the like. Herein, the activation, up-regulation, overexpression, and increase may include showing activity which is not originally present, or showing activity improved compared to the intrinsic activity or activity before modification. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the intrinsic activity, refers to that it is improved compared to the activity and/or concentration (expression level) of a specific polypeptide originally possessed by a parent strain before trait changes or non-modified microorganism.

The enhancement may be achieved by introducing a foreign polypeptide, or enhancement of activity of an intrinsic polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology may be used, but not limited thereto (For example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present application may be
1) an increase of a copy number in cells of a polynucleotide encoding a polypeptide;
2) replacement of a gene expression regulatory region on chromosome encoding a polypeptide with a sequence having strong activity;
3) modification of a base sequence encoding an initiation codon or 5'-UTR area of a gene transcriptome encoding a polypeptide;
4) modification of an amino acid sequence of the polypeptide so that the activity of the polypeptide is enhanced;
5) modification of a polynucleotide sequence encoding the polypeptide so that the activity of the polypeptide is enhanced (for example, modification of a polynucleotide of the polypeptide gene so as to encode a polypeptide modified to enhance the activity of the polypeptide);
6) introduction of a foreign polypeptide showing activity of a polypeptide or a foreign polynucleotide encoding the same;
7) codon optimization of a polynucleotide encoding a polypeptide;
8) modification or chemical modification by analyzing the tertiary structure of a polypeptide and select an exposed site; or
9) a combination of at least 2 selected from the 1) to 8), but not particularly limited thereto.

More specifically, the 1) increase of a copy number in cells of a polynucleotide encoding a polypeptide may be achieved by introduction of a vector which can replicate and function regardless of a host, in which a polynucleotide encoding the corresponding polypeptide is operably linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of a gene transcriptome encoding a polypeptide may be for example, substituting it with a base sequence encoding another initiation codon with a higher polypeptide expression rate compared to the intrinsic initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an intrinsic polynucleotide is increased in a host cell, or codon optimization so that a foreign polynucleotide is optimized in a host cell to achieve transcription and translation.

The 8) modification or chemical modification by analyzing the tertiary structure of a polypeptide and select an exposed site may determine template protein candidates depending on the degree of similarity of sequences by comparing sequence information of a polypeptide to be analyzed, and confirm the structure based on this, and select an exposed site to be modified or chemically modified, and transform or modify it.

Such enhancement of the polypeptide activity, may be that the activity or concentration expression level of the corresponding polypeptide is increased based on the activity or concentration of the polypeptide expressed in a microbial strain which is a wild-type or before modification, or that the amount of products produced from the corresponding polypeptide is increased, but not limited thereto.

### Recombinant vector

Other embodiment provides a recombinant vector comprising the polynucleotide. The recombinant vector may be used as an expression vector of the polypeptide. The recombinant vector may be for inserting the polynucleotide into genome of a host cell or replacing a corresponding gene of the host cell genome.

The vector of the present application may comprise a DNA preparation comprising a base sequence of a polynucleotide encoding the target polypeptide operably linked to an appropriate expression regulatory region (or expression regulatory sequence) so that a target polypeptide is expressed in an appropriate host. The expression regulatory region may comprise a promoter capable of initiating transcription, any operator sequence for regulating such transcription, a sequence encoding an appropriate mRNA ribosome binding site, and a sequence regulating termination of transcription and translation. The vector may replicate or function regardless of host genome, and may be integrated into the genome itself, after transformed into a suitable host cell.

The vector used in the present application is not particularly limited, and any vector known in the art may be used. Examples of the commonly used vector may include plasmids, cosmids, viruses and bacteriophages in a natural state or recombinant state. For example, as the phage vector or cosmid vector, pWE15, M13, MBL3, MBL4, IXII, ASHII, APII, t10, t11, Charon4A, and Charon21A, and the like may be used, and as the plasmid vector, pDZ-based, pBR-based, pUC-based, pBluescriptll-based, pGEM-based, pTZ-based, pCL-based and pET-based and the like may be used. Specifically, pDZ, pDC, pDCM2, pACYC177, pACYC184, pCL, pECCG117, pUC19, pBR322, pMW118, pCC1BAC, pTop33ori vectors and the like may be used.

As one example, a polynucleotide encoding a target polypeptide may be inserted into chromosome through a vector for inserting chromosome into a cell. The insertion of the polynucleotide into the chromosome may be conducted by any method known in the art, for example, homologous recombination, but not limited thereto. It may further comprise a selection marker for confirming whether the chromosome is inserted. The selection marker is to select a cell transformed with a vector, that is, to confirm whether a target nucleic acid molecule is inserted, and markers that confer a selectable phenotype such as drug resistance, auxotrophy, resistance to cytotoxic agents, or expression of surface polypeptides may be used. In an environment treated with a selective agent, only cells expressing a selection marker survive or show other phenotypes, so the transformed cells may be selected.

In the present application, the term, "transformation" refers to introducing a vector comprising a polynucleotide encoding a target polypeptide into a host cell or microorganism to express the polypeptide encoded by the polynucleotide in the host cell. The transformed polynucleotide may comprise all of them, regardless of whether it is inserted and located on chromosome of the host cell or it is located outside the chromosome, as long as the transformed polynucleotide may be expressed in the host cell. In addition, the polynucleotide includes DNA and/or RNA encoding a target polypeptide. The polynucleotide may be introduced in any form, as long as it can be introduced into a host cell and expressed. For example, the polynucleotide may be introduced into a host cell in a form of an expression cassette which is a gene structure comprising all elements required for being expressed by itself. The expression cassette may commonly comprise a promoter, a transcription termination signal, a ribosome binding site, and a translation termination signal, which are operably linked to the polynucleotide. The expression cassette may be in a form of an expression vector capable of self-replication. In addition, the polynucleotide may be introduced into a host cell in its own form and be operably linked to a sequence required for expression in the host cell, but not limited thereto.

In addition, in the above, the term, "operably linked" means that a promoter sequence that enables to initiate and mediate transcription of a polynucleotide encoding the target variant of the present application and the polynucleotide sequence are functionally linked.

### Microorganism

Other aspect provides a microorganism comprising at least one (for example, 1 kind or more, 2 kinds or more, or 1 kind, 2 kinds, or 3 kinds) selected from the group consisting of the variant polypeptide, a polynucleotide encoding (or coding) the polypeptide, and a recombinant vector comprising the polynucleotide. In one embodiment, the microorganism may be a microorganism in which production ability of beta-alanine and/or beta-alanine-derived compounds is increased (or enhanced).

In the present application, the term, "microorganism (or, strain)" includes all of wild-type microorganisms or microorganisms in which genetic modification occurs naturally or artificially, and may be a microorganism comprising genetic modification for production of a targeted polypeptide, protein or product (for example, aspartate 1-decarboxylase), which is a microorganism in which specific mechanism is weakened or intensified due to causes such as that a foreign gene is inserted and/or that activity of an intrinsic gene is enhanced or inactivated.

The microorganism of the present application may be a genetically modified microorganism (for example, recombinant microorganism) through a vector comprising the variant polypeptide or a polynucleotide encoding thereof, but not limited thereto. The vector is as described above.

The microorganism (or strain, recombinant cell) of the present application may be a microorganism having production ability of beta-alanine and/or beta-alanine-derived compounds or having improved production ability (or production) of beta-alanine and/or beta-alanine-derived compounds.

The microorganism of the present application may be a microorganism naturally having production ability of beta-alanine and/or beta-alanine-derived compounds, or a microorganism in which the production ability of beta-alanine and/or beta-alanine-derived compounds is given or improved, compared to a microorganism having no production ability of beta-alanine and/or beta-alanine-derived compounds, but not limited thereto. The microorganism in which the production ability of beta-alanine and/or beta-alanine-derived compounds is given or improved may be a microorganism in which the variant polypeptide having activity of aspartate 1-decarboxylase is introduced.

That the microorganism (or strain, recombinant cell) has increased (or improved) production ability (or production) of beta-alanine and/or beta-alanine-derived compounds, or has production ability of beta-alanine and/or beta-alanine-derived compounds, may mean that the microorganism (or strain, recombinant cell) may have improved production ability of beta-alanine and/or beta-alanine-derived compounds compared to a non-modified microorganism, a cell before recombination, a parent strain, a wild-type strain, or a microorganism in which aspartate 1-decarboxylase derived from other microorganism (for example, wild-type *T. castaneum*) is introduced; or that the production ability of beta-alanine and/or beta-alanine-derived compounds is given differently from a non-modified microorganism, a cell before recombination, a parent strain, and/or a wild-type strain, which have no production ability of beta-alanine and/or beta-alanine-derived compounds.

The microorganism of the present application may be a microorganism comprising any one or more of the variant polypeptide of the present application, a polynucleotide encoding the variant polypeptide of the present application and a vector comprising the polynucleotide of the present application; a microorganism modified to express the variant polypeptide of the present application or the polynucleotide of the present application; a microorganism (for example, recombinant strain) expressing the variant of the present application or the polynucleotide of the present application; or a microorganism (for example, recombinant strain) having activity of the variant polypeptide of the present application, but not limited thereto.

In one embodiment, the microorganism of the present application may be a *Corynebacterium* sp. microorganism. The *Corynebacterium* sp. Microorganism may be at least one selected from the group consisting of *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, and *Corynebacterium flavescens.*

The microorganism may have improved (increased) production ability of beta-alanine and/or beta-alanine-derived compounds, compared to the same kind of non-modified microorganism. In the present application, the term, "non-modified microorganism" does not exclude a strain comprising mutation that can occur naturally in a microorganism, and may mean a wild-type strain or natural strain itself, or a strain before traits are changed due to genetic mutation caused by natural or artificial factors. For example, the non-modified microorganism may mean a strain in which the variant polypeptide of the present application or a polynucleotide encoding the variant polypeptide is not introduced or before it is introduced, according to one embodiment. Otherwise, the "non-modified microorganism" may mean a strain in which aspartate 1-decarboxylase derived from wild-type *T. castaneum* or a polynucleotide encoding thereof is introduced. The "non-modified microorganism" may be interchangeably used with "strain before modification", "microorganism before modification", "non-mutated strain", "non-modified strain", "non-mutated microorganism" or "reference microorganism". In one embodiment, the non-modified microorganism, which is a subject strain for comparing whether the production ability of beta-alanine and/or beta-alanine-derived compounds is increased, may be specifically wild-type *Corynebacterium glutamicum* ATCC13032 strain, or a strain in which panD of wild-type *T. castaneum* is introduced instead of panD of the wild-type *Corynebacterium glutamicum* ATCC13032 strain, but not limited thereto.

The microorganism (or strain, recombinant cell) may include mutation to increase production of beta-alanine and/or beta-alanine-derived compounds additionally, and any location of the mutation and any kind of genes and/or proteins subject to the mutation may be included without limitation, as long as it increases production of beta-alanine and/or beta-alanine-derived compounds. The recombinant cell may be used without limitation as long as it is a cell capable of transformation.

In one specific embodiment, the microorganism may be accession number KCCM13077P.

The beta-alanine-derived compounds may be at least one selected from the group consisting of β-nitropropanoate, β-amino-propionitrile, N-acetyl-β-alanine, L-aspartate, anserine, spermine, carnosine, β-alanyl arginine, quinolinate, pantothenate (or pantothenic acid), malonate semialdehyde, malonate, acetylene-monocarboxylate and the like, but not limited thereto.

In other embodiment, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 100% or more, about 110% or more, about 120% or more, about 130% or more, about 140% or more, or about 150% more, compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

In other embodiment, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 2.0 times or more, about 2.1 times or more, about 2.2 times or more, about 2.3 times or more, about 2.4 times or more, or about 2.5 times or more, compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

In other embodiment, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 1.0 g/L or more, about 1.1 g/L or more, about 1.2 g/L or more, about 1.3 g/L or more, about 1.4 g/L or more or about 1.5 g/L or more, compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

More specifically, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 100%, about 110%, about 120%, about 130%, about 140%, or about 150% (or about 2.0 times, about 2.1 times, about 2.2 times, about 2.3 times, about 2.4 times, or about 2.5 times; or about 1.0 g/L, about 1.1 g/L, about 1.2 g/L, about 1.3 g/L, about 1.4 g/L or about 1.5 g/L), compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

In other embodiment, the microorganism (or strain, recombinant cell) with improved (increased) production ability (or production) of the beta-alanine and/or beta-alanine-derived compounds may have production ability (or production) of beta-alanine and/or beta-alanine-derived compounds (for example, pantothenic acid) increased by about 5% or more, about 10% or more, about 15% or more, about 20% or more, about 25% or more, about 30% or more, about 35% or more, about 40% or more, about 45% or more, about 50% or more, about 55% or more, about 60% or more, about 70% or more, about 80% or more, about 90% or more, about 100% or more, about 110% or more, about 120% or more, 125% or more, about 130% or more, about 140% or more, or about 150% or more, compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

In other embodiment, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 1.05 times or more, about 1.1 times or more, about 1.15 times or more, about 1.2 times or more, about 1.25 times or more, about 1.3 times or more, about 1.35 times or more, about 1.4 times or more, about 1.45 times or more, about 1.5 times or more, about 1.55 times or more, about 1.6 times or more, about 1.7 times or more, about 1.8 times or more, about 1.9 times or more, about 2 times or more, about 2.1 times or more, about 2.2 times or more, about 2.25 times or more, about 2.3 times or more, about 2.4 times or more, or about 2.5 times or more, compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

In other embodiment, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 0.05 g/L or more, about 0.06 g/L or more, about 0.07 g/L or more, about 0.08 g/L or more, about 0.09 g/L or more, about 0.1 g/L or more, about 0.13 g/L or more, about 0.16 g/L or more, about 0.19 g/L or more, about 0.2 g/L or more, about 0.23 g/L or more, about 0.26 g/L or more, about 0.29 g/L or more, about 0.3 g/L or more, about 0.33 g/L or more, about 0.36 g/L or more, about 0.39 g/L or more, about 0.4 g/L or more, about 0.43 g/L or more, about 0.46 g/L or more, about 0.49 g/L or more, about 0.5 g/L or more, about 0.53 g/L or more, about 0.56 g/L or more, about 0.59 g/L or more, about 0.6 g/L or more, about 0.63 g/L or more, about 0.67 g/L or more, about 0.69 g/L or more, about 0.7 g/L or more, about 0.8 g/L or more, about 0.9 g/L or more, or about 1.0 g/L or more, compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

More specifically, the microorganism (or strain, recombinant cell) with improved (increased) production ability (or production) of the beta-alanine and/or beta-alanine-derived compounds may have production ability (or production) of beta-alanine and/or beta-alanine-derived compounds increased by about 10%, about 15%, about 20%, about 25%, about 35%, about 40%, about 55%. about 100%, or about 125% (or, about 1.1 times, about 1.15 times, about 1.2 times, about 1.25 times, about 1.35 times, about 1.4 times, about 1.55 times, about 2 times, or 2.25 times; or about 0.06 g/L, about 0.1 g/L, about 0.13 g/L, about 0.16 g/L, about 0.23 g/L, about 0.26 g/L, about 0.36 g/L, about 0.5 g/L or about 0.6 g/L), compared to a parent strain before mutated, a non-modified microorganism, a microorganism in which aspartate 1-decarboxylase derived from another microorganism is introduced, or a microorganism in which aspartate 1-decarboxylase derived from wild-type *T. castaneum* is introduced, but not limited thereto.

The term, "about" is a range including all of ±0.5, ±0.4, ±0.3, ±0.2, ±0.1 and the like, and includes all numerical values that are in an equivalent or similar range to the numerical value that follows the term about, but not limited thereto.

Other aspect provides a composition for producing beta-alanine and/or beta-alanine-derived compounds comprising the microorganism of the present application, a medium in which the microorganism is cultured, or a combination thereof.

The composition of the present application may further comprise any appropriate excipient commonly used for a composition for producing beta-alanine and/or beta-alanine-derived compounds, and this excipient may be for example, a preservative, wetting agent, dispersant, suspending agent, buffer, stabilizer, or tonicifying agent, or the like, but not limited thereto.

In the composition of the present application, the microorganism (strain), or medium or the like is as described in other aspect above.

Other aspect provides a method for producing beta-alanine and/or beta-alanine-derived compounds, comprising culturing the microorganism of the present application in a medium.

The method for producing beta-alanine and/or beta-alanine-derived compounds of the present application may comprise culturing the microorganism of the present application in a medium. The microorganism of the present application is as described above.

In the present application, "culturing" means growing a microorganism comprising the variant polypeptide of the present application under an appropriately adjusted environmental condition. The culturing process of the present application may be conducted according to an appropriate medium and culturing conditions known in the art. This culturing process may be easily adjusted and used by those skilled in the art depending on the selected strain. Specifically, the culturing may be batch, continuous, and/or fed-batch, but not limited thereto.

In the present application, "medium" refers to a material in which nutrients required for culturing the microorganism of the present application are mixed as main components, and supplies nutrients and growth factors and the like including water which is essential for survival and growth. Specifically, the medium used for culturing the microorganism of the present application and other culturing conditions may be used as long as it is a medium used for culturing a common microorganism without particular limitation, but the microorganism of the present application may be cultured in a common medium containing a suitable carbon source, a nitrogen source, a phosphorus source, an inorganic compound, an amino acid and/or a vitamin and the like by adjusting temperature, pH and the like under an aerobic condition

Specifically, the culture medium for the microorganism of the present application, for example, the *Corynebacterium* sp. strain may be found in the document ["Manual of Methods for General Bacteriology" by the American Society for Bacteriology (Washington D.C., USA, 1981)].

In the present application, as the carbon source, carbohydrates such as glucose, saccharose, lactose, fructose, sucrose, maltose, etc.; sugar alcohols such as mannitol, sorbitol, etc., organic acids such as pyruvate, lactate, citrate, etc.; amino acids such as glutamic acid, methionine, lysine, etc. and the like, may be included. In addition, natural organic nutrients such as starch hydrates, molasses, blackstrap molasses, rice bran, cassava, sugar cane residues, and corn steep liquor may be used, and specifically, carbohydrates such as glucose and sterilized pre-treated molasses (that is, molasses converted with reducing sugars) and the like may be used, and other carbon sources in an appropriate amount may be variously used without limitation. These carbon sources may be used alone or at least two kinds may be used in combination, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; and organic nitrogen sources such as amino acids including glutamic acid, methionine, glutamine and the like, peptone, NZ-amine, meat extract, yeast extract, malt extract, corn steep liquor, casein hydrates, fish or degraded products thereof, defatted soybean cake or degraded products thereof, and the like may be used. These nitrogen sources may be used alone or at least two may be used in combination, but not limited thereto.

As the phosphorus source, potassium phosphate monobasic, potassium phosphate dibasic, or sodium-containing salts corresponding thereto, or the like may be included. As the inorganic compound, sodium chloride, calcium chloride, iron chloride, magnesium sulfate, iron sulfate, manganese sulfate, calcium carbonate and the like may be used, and in addition thereto, amino acids, vitamins, and/or appropriate precursors and the like may be included. These components or precursors may be added in a batch or continuous method. However, it is not limited thereto.

In addition, by adding a compound such as ammonium hydroxide, potassium hydroxide, ammonia, phosphoric acid, sulfuric acid and the like to a medium during culturing of the microorganism of the present application by an appropriate method, the pH of the medium may be adjusted. Furthermore, during culturing, foam generation may be inhibited using an antifoaming agent such as fatty acid polyglycol ester. Moreover, in order to maintain the aerobic state of the medium, oxygen or oxygen-containing gas may be injected into the medium, or in order to maintain the anaerobic and microaerobic state, gas may be not injected or nitrogen, hydrogen or carbon dioxide gas may be injected, but not limited thereto.

The culture temperature in the culturing of the present application may be maintained at 20 to 45°C, specifically, 25 to 40°C, and culturing may be performed for about 10 to 160 hours, but not limited thereto.

The beta-alanine and/or beta-alanine-derived compounds produced by the culturing of the present application may be released to a medium or remain in cells.

The method for producing beta-alanine and/or beta-alanine-derived compounds of the present application may further comprise preparing the microorganism (strain) of the present application, preparing a medium for culturing the microorganism, or a combination thereof (in any order), for example, before the culturing.

The method for producing beta-alanine and/or beta-alanine-derived compounds of the present application may further comprise recovering beta-alanine and/or beta-alanine-derived compounds from a medium (medium in which culture is performed) or microorganism (for example, *Corynebacterium* sp. Strain) according the culturing. The recovering may be further comprised after the culturing.

The recovery may collect targeted beta-alanine and/or beta-alanine-derived compounds using an appropriate method known in the art according to a culture method of the microorganism of the present application, for example, a batch, continuous or fed-batch culture method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, targeted beta-alanine and/or beta-alanine-derived compounds may be recovered from a medium or microorganism.

In addition, the method for producing beta-alanine and/or beta-alanine-derived compounds of the present application may additionally comprise a purification step. The purification may be performed, using an appropriate method known in the art. In one embodiment, when the method for producing beta-alanine and/or beta-alanine-derived compounds of the present application comprises both the recovery step and purification step, the recovery step and purification step may be continuously or non-continuously performed regardless of the order, or may be performed by being integrated as one step, but not limited thereto.

Other aspect provides a use of the microorganism of the present application for using in production of eta-alanine and/or beta-alanine-derived compounds or preparation of a composition for producing beta-alanine and/or beta-alanine-derived compounds. The microorganism of the present application, beta-alanine and/or beta-alanine-derived compounds or the composition for producing beta-alanine and/or beta-alanine-derived compounds is as described above.

### [ADVANTAGEOUS EFFECTS]

The present application provides a variant polypeptide having an activity of aspartate 1-decarboxylase, a microorganism comprising the same, a composition for producing beta-alanine and/or beta-alanine-derived compounds comprising the microorganism, and a method for producing beta-alanine and/or beta-alanine-derived compounds comprising culturing the microorganism, and the microorganism has an excellent production ability of beta-alanine and/or beta-alanine-derived compounds.

### [MODE FOR INVENTION]

Hereinafter, the present invention will be described in more detail by the following examples. However, they are intended to illustrate the present invention only, but the scope of the present invention is not limited by these examples.

### Example 1. Construction of random mutant strain by NTG-based artificial mutation method and selection of panD variant polypeptide (variant)-expressing strain

In the present example, in order to obtain a microbial mutant strain with a much more improved production ability of beta-alanine, mutation was induced using *Corynebacterium glutamicum* ATCC13032 in which a *panD* gene (Aspartate 1-decarboxylase, or PanD protein) derived from *T. castaneum* was introduced (ATCC13032ΔpanD::panD(TC)) as a parent strain.

The ATCC13032 ΔpanD::panD(TC) strain was constructed by the following method. At first, a vector for defecting the panD gene intrinsically present in the *Corynebacterium glutamicum* ATCC13032 was constructed. PCR was performed using the genomic DNA of *Corynebacterium glutamicum* ATCC13032 as a template and primers of SEQ ID NOs: 73 and 74 and SEQ ID NOs: 75 and 76. PCR was performed under conditions of repeating denaturation 95°C, 30 seconds; annealing 55°C, 30 seconds; and polymerization 72°C, 1 minute, 30 times. As a result, gene fragments of 1000 bp from the panD gene upper part and 1000 bp from the panD gene lower part were obtained, respectively, and each amplification product was purified using QIAGEN's PCR Purification kit, and used as an insert DNA fragment for vector construction.

The pDCM2 (Korean Patent No. 2278000) vector treated with restriction enzyme smal and heat-treated at 65°C for 20 minutes and the DNA fragments (gene fragments of 1000 bp from the panD gene upper part and 1000 bp from the panD gene lower part) were cloned using TaKaRa's Infusion Cloning Kit according to the provided manual so that the molar concentration (M) was to be 2:1:1, thereby constructing the vector pDCM2_ΔpanD for defecting the panD gene on chromosome.

In order to prepare the panD gene derived from *T. castaneum,* PCR was performed using the genomic DNA extracted from *T. castaneum* as a template and primers 77 and 78. PCR was performed by repeating denaturation 95°C, 30 seconds; annealing 55°C, 30 seconds; and polymerization 72°C, 1 minute and 30 seconds, 30 times, and as a result, a DNA fragment of 1640 bp was obtained. In order to secure a lysC promoter derived from *Corynebacterium glutamicum*, PCR was performed in the same way as the method of the above example using the genomic DNA of *Corynebacterium glutamicum* as a template and primers 79 and 90 to obtain a DNA fragment. The pDCM2_ΔpanD vector treated with the restriction enzyme smal and then heat-treated at 65°C for 20 minutes and the obtained DNA fragments were cloned using TaKaRa's Infusion Cloning Kit according to the provided manual so that the molar concentration (M) was to be 2:1:1, to construct the vector pDCM2_ΔpanD::panD(TC) for introducing the panD gene derived from *T. castaneum* on the chromosome.

By transforming the constructed vector pDCM2_ΔpanD::panD(TC) into *Corynebacterium glutamicum* ATCC13032 by electroporation, and passing through a secondary cross process, strains (ΔpanD::panD(TC)) in which *E. coli*-derived panD gene was introduced on the chromosome were obtained, respectively. Appropriate substitution of the *E. coli*-derived panD was confirmed using the following primer combination using MASA (Mutant Allele Specific Amplification) PCR method (Takeda et al., Hum. Mutation, 2, 112-117 (1993)). In other words, the primer combination matching *T. castaneum* panD (SEQ ID NOs: 81 and 80 and SEQ ID NOs: 77 and 82) was first determined by selecting the amplified strain, and secondary selection was completed for the *panD* gene sequence of the selected strain by analyzing it using the primer combination of SEQ ID NO: 81 and SEQ ID NO: 82.

The selected *Corynebacterium glutamicum* ATCC13032 ΔpanD::panD(TC) strain was activated by culturing it in an activation medium for 16 hours, and inoculated in a minimum medium sterilized 121 °C at for 15 minutes and cultured for 14 hours, and then e mL of the cultured solution was recovered. The recovered cultured solution was washed with 100 mM citric acid buffer solution (citric buffer), and then NTG (N-Methyl-N'-nitro-N-nitrosoguanidine) was added so that the final concentration was to be 200 mg/L and treated for 20 minutes, and was washed with 100 mM phosphoric acid buffer solution (phosphate buffer). As a result of measuring the death rate by smearing the NTG-treated strain on a minimum medium, the death rate was shown to be 85%. The survived cells were inoculated and cultured in a production medium, and finally, a mutant strain showing the most excellent beta-alanine production ability was selected, and named *Corynebacterium glutamicum* CJVB5-03-1.

The composition of the medium used in the present example is as follows.

### <Activation medium>

Beef extract 1%, polypeptone 1%, sodium chloride 0.5%, yeast extract 1%, agar 2%, pH 7.2

### <Production medium>

Glucose 10%, yeast extract 0.4%, ammonium sulfate 1.5%, potassium phosphate monobasic 0.1%, magnesium sulfate heptahydrate 0.05%, iron sulfate heptahydrate 10 mg/L, manganese sulfate monohydrate 6.7 mg/L, biotin 50 µg/L, thiamine. HCl 100 µg/L, pH 7.2

### <Minimum medium>

Glucose 1.0%, ammonium sulfate 0.4%, magnesium sulfate 0.04%, potassium phosphate monobasic 0.1%, urea 0.1%, thiamine 0.001%, biotin 200 µg/L, agar 2%, pH 7.2

In order to compare the beta-alanine production ability of *Corynebacterium glutamicum* ΔpanD::panD(TC) and the obtained mutant strain *Corynebacterium glutamicum* CJVB5-03-1, the Corynebacterium glutamicum ΔpanD::panD(TC) strain and CJVB5-04 mutant strain were inoculated in a 250 ml corner-baffled flask containing a production medium of 25 ml, respectively, and then cultured with shaking at 200 rpm at 30°C for 48 hours.

The obtained cultured solution was centrifuged at 20,000 rcf for 10 minutes, and then the supernatant was diluted to 1/10 with TDW (triple distilled water), and HPLC analysis was performed, and the concentration of beta-alanine was measured, and the result was shown in the following Table 1.

**[Table 1]**

| | Beta-alanine production ability of NTG-based mutant strain | |
|---|---|---|
| | | Beta-alanine concentration (g/L) |
| ATCC13032ΔpanD ::panD( TC) | | 0.5 |
| CJVB5-03-1 | | 1.2 |

As a result, as shown in Table 1 above, it was confirmed that the *Corynebacterium glutamicum* ATCC13032 ΔpanD::panD(TC) produced beta-alanine at a concentration of 0.5 g/L, and the mutant strain *Corynebacterium glutamicum* CJVB5-03-1 according to the present application produced beta-alanine at a concentration of 1.2 g/L, and the productivity of beta-alanine increased by about 2.4 times (about 140% increase) compared to the parent strain.

Based on the above result, as a result of genome sequencing of genes in the biosynthetic pathway where beta-alanine is synthesized from pyruvate, in the mutant strain *Corynebacterium glutamicum* CJVB5-03-1, random displacement in the *panD* gene derived from *T. castaneum* was confirmed.

Specifically, it was confirmed that the sequence encoding the 139th phenylalanine (F) of the wild-type PanD protein of *T. castaneum* was mutated into a sequence encoding tyrosine (Y). The amino acid sequence of the mutant PanD (the 139th F (phenylalanine) of the wild-type PanD (SEQ ID NO: 51) derived from *T. castaneum* was mutated into Y (tyrosine)) protein was represented by SEQ ID NO: 52.

As a result of the above, it was confirmed that the mutant strain obtained through the random mutation method could produce beta-alanine with high efficiency and high yield.

### Example 2. Confirmation of pantothenic acid production ability of aspartate 1-decarboxylase variant polypeptide

In Example 1, it was confirmed that the beta-alanine production ability of the aspartate 1-decarboxylase variant polypeptide of *T. castaneum* increased. Additionally, in order to confirm the pantothenic acid production ability of the variant polypeptide having aspartate 1-decarboxylase activity of *T. castaneum,* a microorganism with enhanced activity of 3-methyl-2-oxobutanoate hydroxymethyltransferase (PanB) was constructed.

At first, a vector for defecting an intrinsic *panB* gene present in *Corynebacterium glutamicum* ATCC13032 (a gene encoding 3-methyl-2-oxobutanoate hydroxymethyltransferase, or PanB protein) was constructed. PCR was performed using the genomic DNA of *Corynebacterium glutamicum* ATCC13032 as a template and primers of SEQ ID NOs: 41 and 42 and SEQ ID NOs: 43 and 44. PCR was performed under conditions of repeating denaturation 95°C, 30 seconds; annealing 55°C, 30sceonds; and polymerization 72°C, 1 minute, 30 times. As a result, gene fragments of 1,000 bp from the *panB* gene upper part and 1,000 bp from the *panB* gene lower part were obtained, respectively, and each amplification product was purified using QIAGEN's PCR Purification kit, and used as an insert DNA fragment for vector construction.

The pDCM2 (Korean Patent No. 2278000) vector treated with restriction enzyme smal and heat-treated at 65°C for 20 minutes and the DNA fragments (gene fragments of 1,000 bp from the panB gene upper part and 1,000 bp from the panB gene lower part) were cloned using TaKaRa's Infusion Cloning Kit according to the provided manual so that the molar concentration (M) was to be 2:1:1, thereby constructing the vector pDCM2_ΔpanB for defecting the *panB* gene on chromosome.

In order to prepare the panB gene derived from T. castaneum, PCR was performed using the genomic DNA extracted from E. coli K12 wild-type strain (KCTC1116) as a template and primers 47 and 48. PCR was performed by repeating denaturation 95°C, 30 seconds; annealing 55°C, 30 seconds; and polymerization 72°C, 1 minute and 30 seconds, 30 times, and as a result, a DNA fragment of 795 bp was obtained. In order to secure a lysC promoter derived from *Corynebacterium glutamicum,* PCR was performed in the same way as the method of the above example using the genomic DNA of *Corynebacterium glutamicum* as a template and primers 45 and 46 to obtain a DNA fragment. The pDCM2_ΔpanB vector treated with the restriction enzyme smal and then heat-treated at 65°C for 20 minutes and the obtained DNA fragments were cloned using TaKaRa's Infusion Cloning Kit according to the provided manual so that the molar concentration (M) was to be 2:1:1, to construct the vector pDCM2_ΔpanB::panB(EC) for introducing the *panB* gene derived from *E. coli* on the chromosome.

By transforming the constructed vector pDMC2_ΔpanB::panB(EC) into *Corynebacterium glutamicum* ATCC13032 ΔpanD::panD(TC) and CJVB5-03-1, respectively, by electroporation, and passing through a secondary cross process, strains (ΔpanB::panB(EC)) in which *E. coli*-derived *panB* gene was introduced on the chromosome were obtained, respectively. Appropriate substitution of the *E. coli*-derived panB was confirmed using the following primer combination using MASA (Mutant Allele Specific Amplification) PCR method (Takeda et al., Hum. Mutation, 2, 112-117 (1993)). In other words, the primer combination matching the *E. coli panB* gene (SEQ ID Nos: 49 and 46 and SEQ ID Nos: 47 and 50) was first determined by selecting the amplified strain, and the *panB* gene sequence of the selected strain was secondarily confirmed by analyzing it using the primer combination of SEQ ID NO: 49 and SEQ ID NO: 50.

For the constructed strains, in order to confirm the pantothenic acid productivity, the parent strain and the strains were inoculated in a 250 ml corner-baffled flask containing a production medium of 25 ml consisting of the composition as the example, respectively, and then cultured with shaking at 200 rpm at 33 °C for 48 hours to produce pantothenic acid, and the result was shown in Table 2 below.

**[Table 2]**

| Pantothenic acid production ability of NTG-based mutant strain | | |
|---|---|---|
| | Pantothenic acid concentration (q/L) | L-valine concentration (g/L) |
| ATCC13032ΔpanD::panD(T C)ΔanB::anB(EC) | 0.4 | 1.5 |
| CJVB5-03-1 ΔpanB::panB(EC) | 0.9 | 0.7 |

As a result, as shown in Table 2 above, it was confirmed that *Corynebacterium glutamicum* ATCC13032 ΔpanD::panD(TC)ΔpanB::panB(EC) produced pantothenic acid at a concentration of 0.4 g/L, but the *Corynebacterium glutamicum* CJVB5-04 ΔpanB::panB(EC) according to the present application produced pantothenic acid at a concentration of 0.9 g/L, and therefore, the pantothenic acid productivity increased by about 2.25 times (about 125% increase) compared to the parent strain.

The above result means that the aspartate 1-decarboxylase variant polypeptide derived from *T. castaneum* in the present application could produce not only beta-alanine but also pantothenic acid much more efficiently.

### Example 3. Construction of mutant PanD expression vector having activity of aspartate 1-decarboxylase

In order to confirm that mutation of the position corresponding to the 139^{th} position of the PanD protein (wild-type, SEQ ID NO: 51) derived from *T. castaneum* is important for increasing a production ability of beta-alanine, as confirmed in Example 1, a mutant in which phenylalanine at the 139th position of the PanD protein (wild-type, SEQ ID NO: 51) derived from *T. castaneum* was substituted with another amino acid was constructed, and the effect was confirmed.

Using the wild-type *T. castaneum panD* gene as a template and the primer sequence of Table 3, PCR was performed, and DNA fragments encoding aspartate 1-decarboxylase were amplified. The PCR was performed using PfuUltraTM high-reliability DNA polymerase (Stratagene), and was performed under conditions of repeating denaturation 95°C, 30 seconds; annealing 55°C, for 30 seconds; and polymerization 72°C, 1 minute, 30 times. As a result, a DNA fragment of 430 bp in the 5' upstream region and a DNA fragment of 1224 bp in the 3' downstream region were obtained, focusing on a mutation (mutation in a codon encoding the 139th residue of the PanD protein) of a gene encoding aspartate 1-decarboxylase.

In order to secure PLM1 promoter derived from *Corynebacterium glutamicum,* using the genomic DNA of *Corynebacterium glutamicum* (ATCC13032) as a template and primers of SEQ ID NOs: 39 and 40, PCR was performed in the same way as described above, to obtain promoter DNA fragments.

**[Table 3]**

| List of primers for saturated mutagenesis | | |
|---|---|---|
| Mutant plasmid | Substituted amino acid | Primer SEQ ID NO: |
| tcpanD | F139H | SEQ ID NOs: 1, 5 / 6, 4 |
| | F139T | SEQ ID NOs: 1, 7 / 8, 4 |
| | F139A | SEQ ID NOs: 1, 9 / 10, 4 |
| | F139S | SEQ ID NOs: 1, 15 / 16, 4 |
| | F139L | SEQ ID NOs: 1, 17 / 18, 4 |
| | F139I | SEQ ID NOs: 1, 19 / 20, 4 |
| | F139V | SEQ ID NOs: 1, 25 / 26, 4 |
| | F139D | SEQ ID NOs: 1, 29 / 30, 4 |
| | F139G | SEQ ID NOs: 1, 33 / 34, 4 |
| | F139Y | SEQ ID NOs: 1, 83 / 84, 4 |
| | WT (SEQ ID NO: 51) | SEQ ID NOs: 1, 4 |

The pECCG117 (Korean Patent No. 10-0057684) vector treated with restriction enzyme BamHI and heat-treated at 65°C for 20 minutes and the DNA fragments (each panD, PLM1 promoter) were cloned using TaKaRa's Infusion Cloning Kit according to the provided manual so that the molar concentration (M) was to be 2:1:1:1 (pECCG117 vector: panD upstream region : panD downstream region : promoter), thereby obtaining plasmids, and names of 11 kinds of the plasmids obtained above and information of introduced genes were presented in Table 4.

**[Table 4]**

| 139th amino acid mutant vector list | | |
|---|---|---|
| Mutation position | Amino acid substitution | Mutant plasmid constructed to induce amino acid substitution |
| 139th amino acid residue of wild-type tcpanD (SEQ ID NO: 51) | F139H | pECCG117-panD(F139H) |
| | F139T | pECCG117-panD(F139T) |
| | F139A | pECCG117-panD(F139A) |
| | F139S | pECCG117-panD(F139H) |
| | F139L | pECCG117-panD(F139L) |
| | F139I | pECCG117-panD(F139I) |
| | F139V | pECCG117-panD(F139V) |
| | F139D | pECCG117-panD(F139D) |
| | F139G | pECCG117-panD(F139G) |
| | F139Y | pECCG117-panD(F139Y) |
| | WT (SEQ ID NO: 51) | pECCG117-panD(WT) |

### Example 4. Evaluation of pantothenic acid production ability of mutated PanD

After introducing 19 kinds of the mutant plasmids constructed in Example 3 and pECCG117-panD(WT) into the *Corynebacterium glutamicum* ATCC13032 ΔpanB::panB(EC) strain by the electric pulse method and then smearing in a selection medium containing kanamycin of 25 mg/L, each transformed strain was obtained. After that, flask evaluation was carried out by the same method as Example 2, and the result was as the following Table 5.

**[Table 5]**

| Pantothenic acid production ability of saturated mutagenesis-introduced strains | | | | |
|---|---|---|---|---|
| Strain | Pantothenic acid (g/L) | | | |
| | Batch 1 | Batch 2 | Batch 3 | |
| ATCC 13032 ΔpanB ::panB(EC) | 0.0 | 0.0 | 0.0 | |
| ATCC 13032 ΔpanB ::panB(EC)pECCG117-panD(F139T) | 0.9 | 0.9 | 1.0 | |
| ATCC 13032 ΔpanB ::panB(EC)pECCG117-panD(F139A) | 0.8 | 0.7 | 0.8 | |
| ATCC 13032 ΔpanB ::panB(EC)pECCG117-panD(F139S) | 0.9 | 0.9 | 0.9 | |
| ATCC 13032 ΔpanB ::panB(EC)pECCG117-panD(F139L) | 0.7 | 0.8 | 0.7 | |
| ATCC 13032 ΔpanB ::panB(EC)pECCG117-panD(F139I) | 1 | 1 | 0.8 | |
| ATCC 13032 ΔpanB ::panB(EC)pECCG117-panD(F139V) | 0.7 | 0.8 | 0.9 | |
| ATCC 13032 ΔpanB ::panB(EC)pECCG117-panD(F139D) | 0.7 | 0.8 | 0.7 | |
| ATCC 13032 ΔpanB ::panB(EC)pECCG117-panD(F139G) | 0.9 | 0.7 | 0.9 | |
| ATCC 13032 ΔpanB ::panB(EC)pECCG117-panD(F139H) | 1.1 | 1 | 1 | |
| ATCC 13032 ΔpanB ::panB(EC)pECCG117-panD(F139Y) | 1.4 | 1.3 | 1.3 | |
| ATCC 13032 ΔpanB ::panB(EC)pECCG117-panD(WT) | 0.7 | 0.7 | 0.6 | |

As could be seen in Table 5 above, all of the strain in which the wild-type panD was introduced (ATCC 13032 ΔpanB ::panB(EC) pECCG117-panD(WT)) and the strains in which the mutant panD was introduced had an increased production activity of pantothenic acid.

In particular, it was confirmed that a total of 10 kinds of mutant strains (F139T, F139A, F139S, F139L, F139I, F139V, F139D, F139G, F139H, F139Y) including a mutant strain expressing a mutant PanD in which the 139th amino acid of the wild-type PanD protein was mutated to tyrosine produced pantothenic acid at a higher level than a strain expressing the wild-type PanD protein, respectively, and among them, one of the mutant strain (F139Y) produced pantothenic acid twice or more than the wild type. As a result, it could be confirmed that the 139th position of the PanD protein derived from T. *castaneum* is an important position for activity, and it could be confirmed that it is an important position that affects the production ability of pantothenic acid, when the position was substituted with another amino acid.

In the present example, ATCC 13032 ΔpanB ::panB(EC) pECCG117-panD(F139Y) strain (named *Corynebacterium glutamicum* CV03-5004), which was confirmed to have the most excellent pantothenic acid production ability, was deposited at Korean Culture Center of Microorganisms located in Hongje-dong, Seodaemun-gu, Seoul, Republic of Korea, on November 23, 2021 and given the accession number of KCCM13077P.

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

### [ACCESSION NUMBER]

Name of Depository Authority: Korean Culture Center of Microorganisms
Accession number: KCCM13077P
Date of deposit: 20211123

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CV03-5004 | Accession number given by the
INTERNATIONAL DEPOSITARY AUTHORITY:
KCCM13077P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 23. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 23. 2021.

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS [?]
Yurim Bldg., 45, Hongjenae-2ga-gil, Seodaemun-gu, Seoul, 03641, Korea. Tel: 82-2-391-0950, 396-0950 Fax: 82-2-392-2859

## Claims

1. A variant polypeptide having an activity of aspartate 1-decarboxylase, in which an amino acid corresponding to the 139th residue of an amino acid sequence of SEQ ID NO: 51 is substituted with another amino acid.

2. The polypeptide according to claim 1, wherein the amino acid corresponding to the 139th residue of an amino acid sequence of SEQ ID NO: 51 is substituted with tryptophan, histidine, tyrosine, alanine, cysteine, proline, serine, leucine, isoleucine, arginine, lysine, valine, methionine, aspartic acid, glutamic acid, glycine, asparagine, glutamine, or tyrosine.

3. The polypeptide according to claim 1, wherein the amino acid corresponding to the 139th residue of an amino acid sequence of SEQ ID NO: 51 is substituted with threonine, alanine, serine, leucine, isoleucine, valine, aspartic acid, glycine, histidine, or tyrosine.

4. The polypeptide according to claim 1, wherein the polypeptide has sequence identity of 70% or more and less than 100% to an amino acid sequence of SEQ ID NO: 51.

5. A polynucleotide encoding the polypeptide of any one of claims 1 to 4.

6. A microorganism comprising a variant polypeptide having an activity of aspartate 1-decarboxylase, in which an amino acid corresponding to the 139th residue of an amino acid sequence of SEQ ID NO: 51 is substituted with another amino acid, or a polynucleotide encoding the same.

7. The microorganism according to claim 6, wherein the microorganism has an increased production ability of beta-alanine or beta-alanine-derived compound.

8. The microorganism according to claim 6, wherein the microorganism is a *Corynebacterium* sp. microorganism.

9. The microorganism according to claim 8, wherein the *Corynebacterium* sp. microorganism is *Corynebacterium glutamicum.*

10. A composition for producing beta-alanine or beta-alanine-derived compound,
comprising a microorganism comprising a variant polypeptide having an activity of aspartate 1-decarboxylase, in which an amino acid corresponding to the 139th residue of an amino acid sequence of SEQ ID NO: 51 is substituted with another amino acid or a polynucleotide encoding the same.

11. A method for producing beta-alanine or beta-alanine-derived compound, comprising culturing a microorganism comprising a variant polypeptide having an activity of aspartate 1-decarboxylase, in which an amino acid corresponding to the 139th residue of an amino acid sequence of SEQ ID NO: 51 is substituted with another amino acid or a polynucleotide encoding the same in a medium.

12. The method for producing beta-alanine or beta-alanine-derived compounds according to claim 11, further comprising recovering beta-alanine or beta-alanine-derived compound from the cultured microorganism, medium, or all of them, after the culturing.
